# EUROPEAN PATENT SPECIFICATION

(11) **EP 4 000 565 B1**
(45) Date of publication and mention of the grant of the patent: **09.12.2026**
(21) Application number: 21212738.5
(22) Date of filing: 17.03.2014
(51) Int. Cl.: A61F 2/44, A61F 2/28

(54) **MEDICAL IMPLANT FOR FIXATION AND INTEGRATION WITH HARD TISSUE**
MEDIZINISCHES IMPLANTAT ZUR FIXIERUNG UND INTEGRATION MIT HARTGEWEBE
IMPLANT MÉDICAL DE FIXATION ET D'INTÉGRATION À UN TISSU DUR

(30) Priority: 15.03.2013 US 201361794341 P
(43) Date of publication of application: 25.05.2022
(62) Document number(s) of the earlier application(s) in accordance with Art. 76 EPC: 14765130.1 / 2 967 903
(73) Proprietor: Lifenet Health, Virginia Beach, VA 23453 (US)
(72) Inventor: CHEN, Jingsong, Virginia Beach, 23453 (US); PHELPS, Dennis, Virginia Beach, 23453 (US); PRICHARD, Andy, Virginia Beach, 23453 (US); KEMPER, Nathan, Virginia Beach, 23453 (US); BREWER, Thomas, Virginia Beach, 23453 (US)
(74) Representative: Scheele, Friedrich

(56) References cited:
- EP-A1- 2 047 825
- WO-A2-2007/079021
- WO-A2-2007/127771
- US-A1- 2003 149 484
- US-A1- 2003 187 436
- US-A1- 2011 035 007

## Description

### FIELD OF THE INVENTION

This invention relates generally to medical implants useful for fixation and integration with hard tissue e.g., bone tissue and cartilage tissue. More particularly, the invention relates to medical implants, including spinal implants and bone grafts, which are used in hard tissue fusion without the use of separate fixation or anchoring structures, such as supporting plates or screws. The medical implants of the invention, which may be composite or monolithic, include one or more fixation mechanisms as an integral component. The fixation mechanisms include one or more sharp protrusions configured to penetrate and become lodged into hard tissue at an integration site to provide further support and stabilization. For example, a sharp protrusion may penetrate and become lodged into a vertebral body to provide support and stabilization between fused vertebrae.

### BACKGROUND OF THE INVENTION

The typical vertebra has a thick anterior bone mass called the vertebral body, with a neural vertebral arch that arises from the posterior surface of the vertebral body. The central portions of adjacent vertebrae are supported by intervertebral discs. The spinal disc and/or vertebral bodies may be displaced or damaged due to trauma, disease, degenerative defects, or wear over an extended period of time. One result of this displacement or damage to a spinal disc or vertebral body may be chronic back pain. In many cases, to alleviate back pain from degenerated of herniated discs, the disc is removed along with all or part of at least one neighboring vertebrae and is replaced by an implant that promotes fusion of the remaining bony anatomy. However, the success or failure of spinal fusion may depend upon several factors. For instance, the spacer or implant used to fill the space left by the removed disc and bony anatomy must be sufficiently strong to support the spine under a wide range of loading conditions. The spacer should also be configured so that it likely to remain in place once it has been positioned in the spine by the surgeon. Additionally, the material used for the spacer should be biocompatible and should promote bone growth and integration.

An additional support system, such as a system including plates and/or screws, is typically used in combination with spacers to further stabilize the spine during the fusion process. These devices, commonly referred to as bone fixation plating systems, typically include one or more plates and screws for aligning and holding vertebrae in a fixed position with respect to one another. Plating systems that are independent of the spacers often present additional failure modes for the spinal fusion process. For example, the screws may loosen over time, or the hardware may fail due to other means, such as breakage.

Similar problems arise with medical implants which integrate with hard tissue at other locations in the body. Such medical implants may be used, for example, in a knee, shoulder, elbow, wrist, ankle, hip, or finger.

Given the problems discussed above, there is a need for a spine stabilization system that promotes fusion of adjacent vertebrae while at the same time providing stabilization of the spinal area where fusion occurs. Thus, there is a need for a spinal implant or bone graft that addresses the problems associated with using separate spacers and stabilization systems.

More broadly, there is a need for medical implants used without separate stabilization systems that are useful for fixation and integration with hard tissue at other locations in the body. Accordingly, one aspect of the invention is to provide such an implant and a method (not claimed) for using the same. Other embodiments will be apparent from the following description of the invention.

US 2011/035007 illustrates for example a stand-alone interbody fixation system having a cage, anterior fixation blade and posterior fixation blade. The cage includes an annular side wall with an open interior and upper and lower surfaces, the cage being configured to fit between end plates of adjacent vertebrae. The anterior fixation blade includes an anterior alignment boss with two opposing outward extending anterior blades with end plate penetrating tips configured to fit within the open interior of the cage. The posterior fixation blade includes a posterior alignment boss with two opposing outward extending posterior blades with end plate penetrating tips configured to fit within the open interior of the cage. The anterior alignment boss and posterior alignment boss being rotatably coupled to each other and with a first opening and a second opening in the annular side wall opposite the first opening. The anterior and posterior fixation blades are counter-rotating blades and the anterior alignment boss and posterior alignment boss are configured to receive or engage a blade activation tool having an anterior engagement portion and a posterior engagement portion configured to rotate the anterior and posterior fixation blades from a stowed position to a deployed condition.

WO 2007/079021 A2 relates to a spinal fixation and fusion device that includes a housing with leading deep surface conforming to the posterior aspect of the intervertebral disk and trailing outer surface conforming to the anterior surface of the disk, weight bearing sides and the top and bottom surfaces with plurality of openings enabling ingrowths of bone. The device further includes a shaft running from the center of the deep surface and perpendicular to the deep surface of the housing to the center of the outer surface of the housing and affixed at least to the deep surface of the housing, and a flat metal member threaded onto the shaft with sharp leading edge which upon clockwise or counterclockwise rotation about the axis of the shaft will break the endplate, hook into the vertebra and rigidly secure the vertebra to the entire device preventing separation of the vertebra from the device during spinal motion.

WO 2007/127771 A2 provides spinal implants and systems which may be used for fixing a portion of the spine, such as the cervical spine, to allow correction or healing. One such spinal implant includes a body having at least one cavity therein. A barb is disposed within the cavity, with the barb adapted to rotate about a support member between a retracted position and a deployed position. In this manner, the barb may be selectively moved between desired positions, including the retracted and deployed positions. The retracted position may be used during insertion, removal, or repositioning of the implant between two vertebral bodies.

EP 2 047 825 A1 concerns a system, device and method are disclosed for minimally invasive lateral intervertebral fixation.

US 2003/187436 A1 relates to an interbody spinal stabilization method and an interbody spinal stabilization cage.

US 2003/149484 A1 discloses an interbody spinal fusion implant for implantation at least in part within and across the generally restored height of a disc space between two adjacent vertebral bodies of a human spine having an external housing and a substantially hollow internal rotatable member having bone engaging projections that are deployable through the housing to penetrably engage the adjacent vertebral bodies.

### SUMMARY OF THE INVENTION

According to one aspect of the invention, a medical implant for fixation and integration with hard tissue e.g., bone tissue and cartilage tissue is provided at an integration site. The integration site may include, for example, a vertebra including a vertebral body, a knee, a shoulder, an ankle, an elbow, a wrist, a hip or a finger. Certain embodiments of the invention relate to integration at the vertebrae. Such a process may be further described as spine fixation, anterior column fusion, spinous process fusion, and facets fusion.

According to one aspect of the invention, a medical implant is provided for implantation into a host. More specifically, a compound medical implant is provided for fixation and integration with hard tissue, the implant comprising one or more rotational fixation mechanisms substantially centered on an axis e.g., a pin or a rod; and one or more sharp protrusions attached to each of the one or more rotational fixation mechanisms, each sharp protrusion designed to penetrate and become lodged into hard tissue at an integration site. While the implantation methods described herein do not form part of the invention, they are disclosed as they represent useful background for understanding the invention.

The invention is defined in the independent claim. Further advantageous embodiments are defined in the dependent claims.

### BRIEF DESCRIPTION OF THE DRAWINGS

The following examples shown in Figs 1 to 11, and 17 to 39 are not according to the invention and are present for illustration purposes only. The scope of the protection of the invention is solely determined by the appended set of claims.
- FIG. 1: presents a perspective view of a disassembled composite spinal implant not according to the invention.
- FIG. 2: presents another perspective view of a disassembled composite spinal implant not according to the invention.
- FIG. 3: presents another perspective view of a disassembled composite spinal implant not according to the invention.
- FIG. 4: presents another perspective view of a disassembled composite spinal implant not according to the invention.
- FIGS. 5A, 5B and 5C: present different views of an assembled stand-alone composite spinal implant not according to the invention.
- FIG. 6: presents a perspective view of a disassembled textured spinal implant not according to the invention.
- FIG. 7: presents another perspective view of a disassembled textured spinal implant not according to the invention.
- FIG. 8: presents a perspective view of an assembled textured spinal implant not according to the invention.
- FIG. 9: presents a perspective view of an assembled spinal bone graft not according to the invention.
- FIG. 10: presents a perspective view of an assembled spinal bone graft not according to the invention.
- FIG. 11: presents a perspective view of an assembled spinal bone graft according to the present invention.
- FIG. 12: presents a perspective view of a textured spinal implant according to one embodiment of the invention, including a double angled sharp protrusion and a screw used to urge a rotational fixation mechanism to implant the sharp protrusion into hard tissue.
- FIG. 13: presents a perspective view of a textured spinal implant according to one embodiment of the invention.
- FIG. 14: presents a side view of a textured spinal implant according to one embodiment of the invention.
- FIG. 15: presents a side view of a textured spinal implant according to one embodiment of the invention.
- FIG. 16: presents a perspective view of a textured spinal implant according to one embodiment of the invention. Several of the parts are shown disassembled.
- FIG. 17: presents a perspective view of a composite spinal implant not according to the invention.
- FIG. 18: presents a perspective view of a composite spinal implant not according to the invention.
- FIG. 19: presents a side view of a composite spinal implant not according to the invention.
- FIG. 20: presents a side view of a composite spinal implant not according to the invention.
- FIG. 21: presents a cut away side view of a composite spinal implant not according to the invention.
- FIG. 22: presents a cut away side view of a composite spinal implant not according to the invention.
- FIG. 23: presents a side view of composite medical implant according to one embodiment of the invention.
- FIG. 24: presents a side view of composite medical implant according to one embodiment of the invention.
- FIG. 25: presents a side cut away view of composite medical implant according to one embodiment of the invention.
- FIG. 26: presents a side cut away view of composite medical implant according to one embodiment of the invention.
- FIG. 27: presents a perspective view of composite medical implant according to one embodiment of the invention.
- FIG. 28: presents a perspective view of composite medical implant according to one embodiment of the invention.
- FIG. 29: presents a side cut away view of composite medical implant according to one embodiment of the invention.
- FIG. 30: presents a side cut away view of composite medical implant according to one embodiment of the invention.
- FIG. 31: presents a side cut away view of composite medical implant according to one embodiment of the invention.
- FIG. 32: presents a perspective view of composite medical implant according to one embodiment of the invention.
- FIG. 33: presents a perspective view of composite medical implant according to one embodiment of the invention.
- FIG. 34: presents a perspective view of a composite medical implant not according to the invention.
- FIG. 35: presents a cut away side view of a composite medical implant not according to the invention.
- FIG. 36: presents a cut away side view of a composite medical implant not according to the invention.
- FIG. 37: presents a top view of a composite medical implant not according to the invention.
- FIG. 38: presents a side view of a composite medical implant not according to the invention.
- FIG. 39: presents a side view of a composite medical implant not according to the invention.

### DETAILED DESCRIPTION OF THE INVENTION

The present invention provides medical implants, including spinal implants and spinal bone grafts, useful for fixation and integration with hard tissue e.g., bone or cartilage tissue. Importantly, the medical implants may be used in the absence of separate fixation or anchoring structures, such as supporting plates or screws. For example, in one embodiment, the invention provides for spinal implants to fuse together adjacent vertebral bodies while maintaining the natural curve of the spine and the proper spacing between the adjacent vertebral bodies. The implant has a size and a geometry that enables it to be efficiently installed and to remain securely placed between adjacent vertebral bodies until healing and fusion take place. Moreover, the spinal implants and bone grafts may be used in spinal fusion without the further use of stabilization structures, e.g., supporting plates or screws.

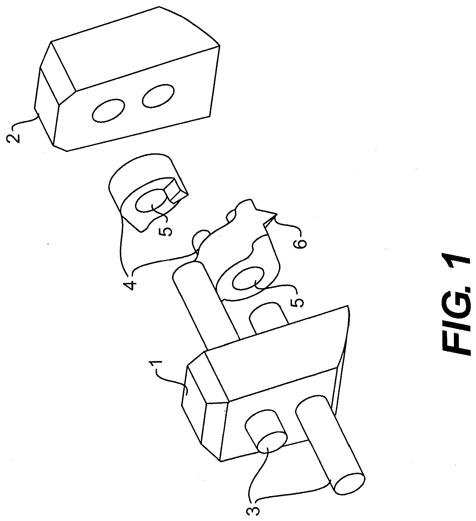

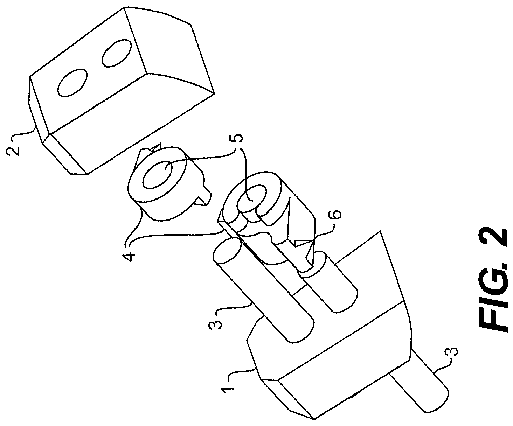

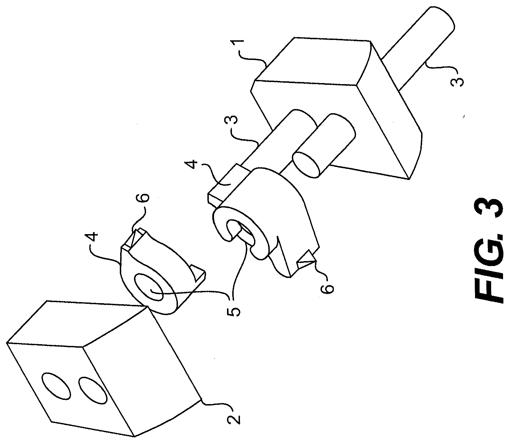

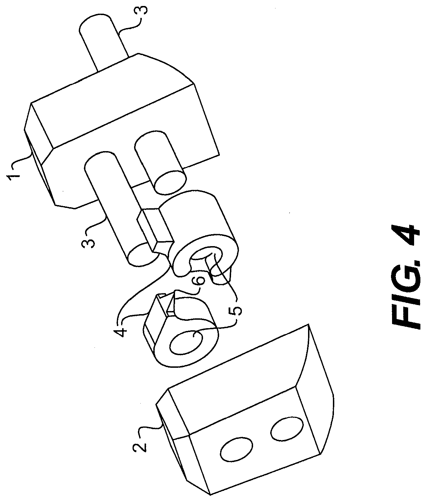

Referring more specifically to the drawings, FIGS. 1-4 provide varying perspective views of a disassembled composite spinal implant . A first portion 1 and a second portion 2 are provided, each having an inferior and a superior surface. One or more biocompatible pins 3 may be used to hold together the first and second portion. One or more rotational fixation mechanisms 4 may be located between the first portion and the second portion. The rotational fixation mechanisms may comprise an opening 5 through which the one or more pins traverse to secure the rotational fixation mechanisms in place. The rotational fixation mechanisms include at least one sharp protrusion 6 configured to penetrate a vertebra. This is useful for providing stabilization of the spinal area where fusion occurs, and the resultant spinal bone graft may be used without additional stabilizing structures, such as supporting plates or screws.

The sharp protrusion 6 may have different cross-sectional profiles. For example, in one embodiment, the sharp protrusion has a pyramidal shaped cross-section. In another embodiment, the sharp protrusion has a circular, oval, or knife-like cross-section.

The various components of the composite implant, independently, may be made of different materials, natural and synthetic, including, but not limited to, cortical bone, biocompatible metals, ceramics and biocompatible polymer. Examples of suitable metals include but are not limited to titanium, aluminum, stainless steel, or alloys e.g., Ti6AI-4V. Ceramics may include but are not limited to glass-ceramics and apatite-wollastonite ceramics. Polymers may include but are not limited to polysiloxane modified styrene-ethylene/butylene block copolymer or polyether ether ketone PEEK.

The implants may further comprise bone graft substitutes, including but not limited to hydroxyapatite, tricalcium phosphate TCP and osteoinductive growth factors bone morphogenetic protein, BMP, and transforming growth factor, TGFβ, and/or cells. The cells may be differentiated cells, progenitor cells, or stem cells. The stem cells may be adult stem cells or pluripotent stem cells. The progenitor cells or the adult stem cells may be derived from placenta, bone marrow, adipose tissue, blood vessel, amniotic fluid, synovial fluid, synovial membrane, pericardium, periosteum, endosteum, dura, peripheral blood, umbilical blood, menstrual blood, baby teeth, nucleus pulposus, brain, skin, hair follicle, intestinal crypt, neural tissue, or muscle. The growth factor can be truncated growth factors as described in US Published Application Nos. US2013/0090290, US2013/0196910A1 and US2013/0196909A1, and WO2013033680 .

In one embodiment, all of the components i.e., the first and second portion, the one or more pins and the one or more rotational fixation mechanisms are made of cortical bone.

In another embodiment, the first portion, the second portion and the pins are made of cortical bone.

In one embodiment, the rotational fixation mechanism is made of a biocompatible metal.

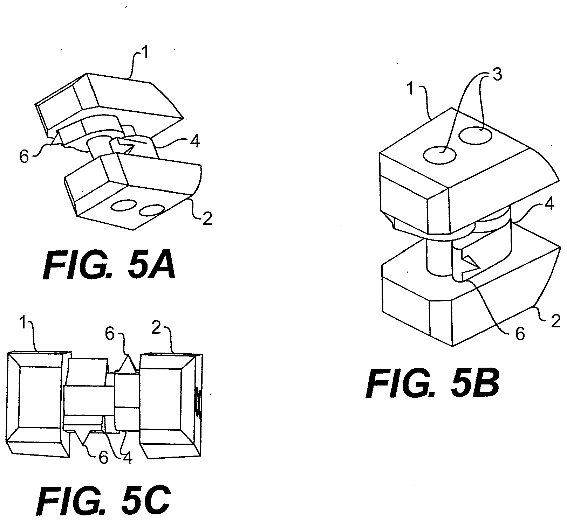

FIGS. 5A, 5B and 5C provide views of the assembled composite spinal implant. The one or more rotational fixation mechanisms may include at least one sharp protrusion. In one embodiment, the sharp protrusion as a length ranging from 0.1 mm to about 4 cm. In one embodiment, the sharp protrusion may have a length ranging from about 0.1 mm to about 1.5 cm. In another embodiment, the sharp protrusion has a length ranging from about 0.5 mm to about 0.8 cm.

The rotational fixation mechanisms may rotate about the pin prior to final assembly. At final assembly or when the rotational fixation mechanisms are implanted into a host during surgery, the rotational fixation mechanisms are locked in place so that they may stabilize the spinal implant when it is positioned in the spine.

In an embodiment, the fully assembled spinal implant as discussed above has built in void space between the pins 3 and the rotational fixation mechanisms 4. This has a number of functions, including allowing space for to promote fusion between the two vertebral bodies or adjacent hard tissue.

In one embodiment, the at least one sharp protrusion penetrates a vertebrae at an angle ranging from about 15° to about 90° with respect to a planar face of the vertebral bodies. In another embodiment, the sharp protrusion may penetrate a vertebral body at an angle ranging from about 30° to about 60°. In another embodiment, the sharp protrusion may penetrate a vertebral body at an angle from about 40° to 50°.

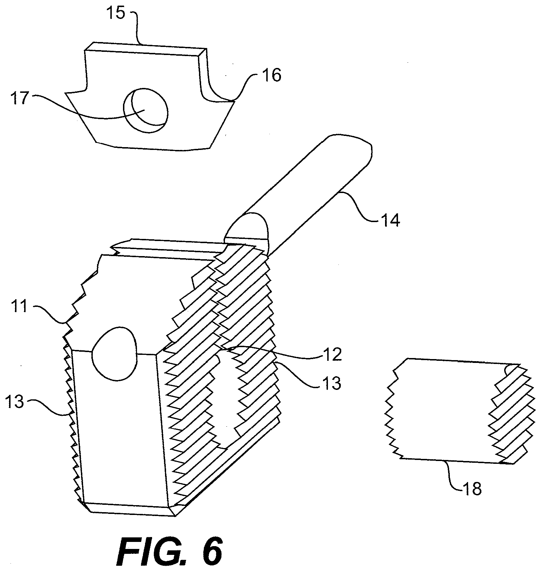

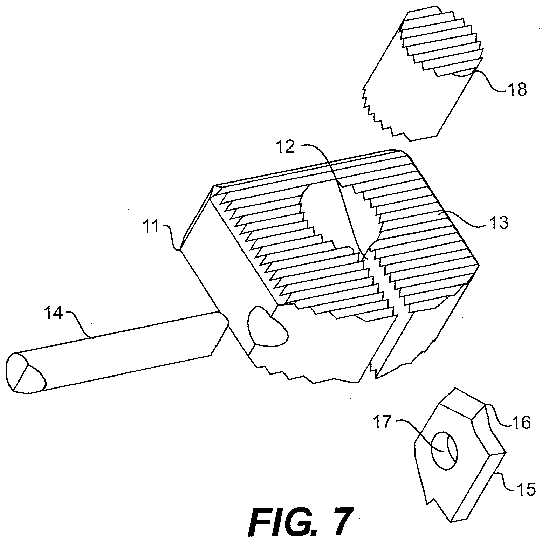

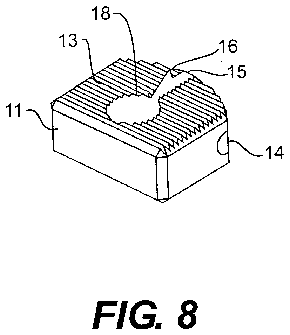

FIGS. 6a, 6b and 7 provide views of a disassembled textured spinal implant. FIG. 8 provides a view of an assembled textured spinal implant. The textured spinal implant includes base member 11, a biocompatible pin 14, and one or more rotational fixation mechanisms 15. Base member 11 includes a key-shaped through-section or void space 12 and one or more textured surfaces 13 configured to contact a portion of the host bone. A biocompatible pin 14 is approximately parallel to the surfaces and perpendicular to the key-shaped through-section at its narrow portion. The one or more rotational fixation mechanisms 15 have an opening 17 through which the pin traverses, the one or more rotational fixation mechanisms including at least one sharp protrusion 16 configured to penetrate a vertebral body to provide positional stability. The at least one sharp protrusion exceeds the height of the textured surface.

In one embodiment, the textured spinal implant comprises two rotational fixation mechanisms, one having a sharp protrusion configured to penetrate and become lodged into the vertebral body above the implant and the other having a sharp protrusion configured to penetrate and become lodged into the vertebral body below the implant, thus providing fixation and positional stability of the implant between the two vertebral bodies. In one embodiment, the two rotational fixation mechanisms may be connected to one another. In another embodiment, the two rotational fixation mechanisms may be configured to act independently of one another to provide positional stability of the implant between two vertebrae.

The remaining portion of the void space i.e., the portion of the void space remaining after the rotational fixation mechanism is located in place may be filled 18 with cancellous bone or other osteoconductive materials e.g., hydroxyapatite, tricalcium phosphate TCP with or without osteoinnductive growth factors bone morphogenetic protein, BMP, and transforming growth factor, TGFβ, and/or cells.

In one embodiment, the sharp protrusion has a length ranging from 0.1 mm to about 4 cm. In one embodiment, the sharp protrusion may have a length ranging from about 0.1 mm to about 1.5 cm. In another embodiment, the sharp protrusion has a length ranging from about 0.5 mm to about 0.8 cm.

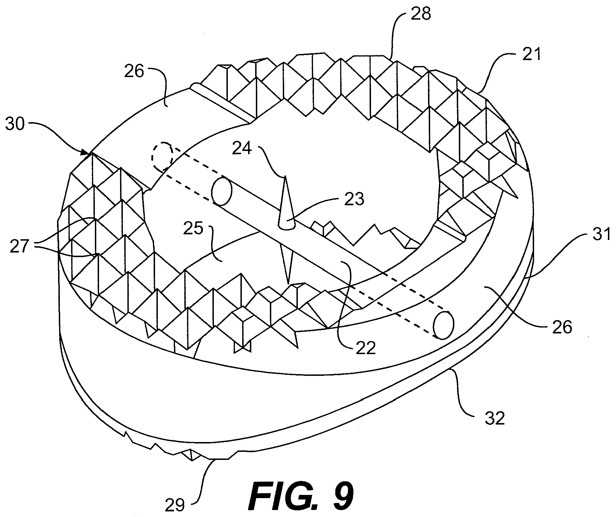

FIG. 9 provides a view of an assembled spinal implant for implantation into a host. The graft of this embodiment is a solid annular ring-like body 21 having a substantially central through hole or void space 25. The body has opposed superior 28 and inferior 29 surfaces and anterior and posterior ends 30, 31. At least a portion of the superior and inferior surfaces includes a plurality of bone engaging protrusions 27. These protrusions may be discrete. The superior and inferior surfaces each may include one or more zones or regions 26 that are free of any bone-engaging protrusions. The posterior end of the implant may include a tip 32 that is formed of converging portions of the superior and inferior surfaces.

One or more biocompatible pins 22 may traverse the central opening in the ring-like body. One or more rotational fixation mechanisms 23 are located within the central opening on the one or more pins, the one or more rotational fixation mechanisms including at least one sharp protrusion 24 configured to penetrate a vertebral body. The sharp protrusion may have different cross-sectional profiles. In one embodiment, the pin is fully rotatable.

In one embodiment, the sharp protrusion has a pyramidal shaped cross-section. In one embodiment, the sharp protrusion has a pyramidal shaped cross-section. In another embodiment, the sharp protrusion has a circular, oval, or knife-like cross-section.

The body of the spinal graft of this embodiment may be made of cortical bone and thus may be referred to as a bone graft.

In another embodiment, the body of the spinal graft of this embodiment may be made of a biocompatible metal e.g., titanium or Ti6AI-4V, a ceramic e.g., glass-ceramics and apatite-wollastonite ceramics or a biocompatible polymer e.g., PEEK or polysiloxane modified styrene-ethylene/butylene block copolymer.

The various other components of this embodiment, independently, may be made of different materials, including, but not limited to, cortical bone, a biocompatible metal and a biocompatible polymer such as polyether ether ketone PEEK.

In one embodiment, the rotational fixation mechanism is made of a biocompatible metal.

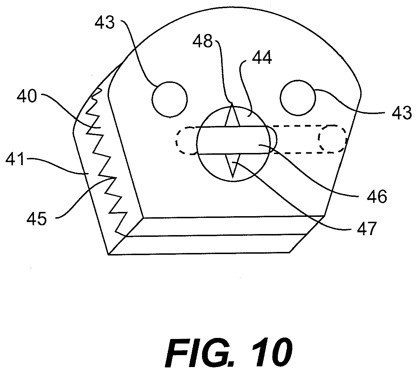

FIG. 10 illustrates a perspective view of a wedge composite bone graft flattened curved wedge composite bone graft. The flattened curved wedge composite bone graft includes first and second cortical bone portions 40, 41 held together by two cortical bone pins 43 to form a pinned graft unit, and the pinned graft unit having an void space 44 disposed therethrough located between pins 43. The cortical bone portions 40, 41 are patterned with grooves 45 running in direction to provide an interlocking fit between the bone portions.

One or more biocompatible pins 46 may traverse the central void space in the cortical bone ring-like body. One or more rotational fixation mechanisms 47 are located within the central void space on the one or more pins, the one or more rotational fixation mechanisms including at least one sharp protrusion 48 configured to penetrate a vertebral body.

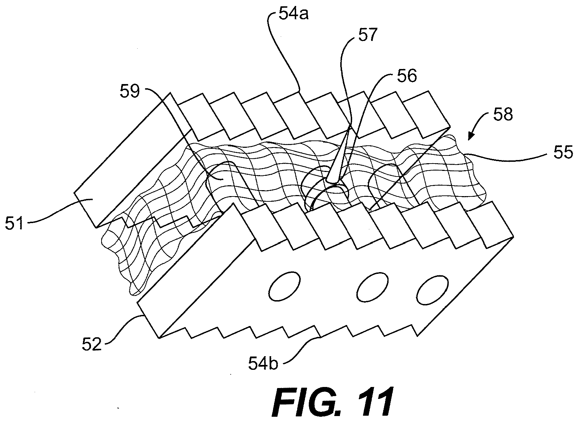

FIG. 11 shows a perspective view of a rectangular block composite bone graft, including a first cortical bone portion 51, a second cortical bone portion 52, a void space 58 disposed between cortical bone portions 51 and 52, through-holes 53, and biocompatible pins e.g., cortical bone pins 59. The void space 58 includes one or more therapeutically beneficial substances 55. The composite bone graft includes opposing textured surfaces 54a and 54b provided perpendicular to the interfaces of the bone portions 51 and 52, with the therapeutically beneficial substance 55, and defining a saw-tooth-like pattern. The therapeutically beneficial substances may include, but are not limited to, cancellous bone or other osteoconductive materials e.g., hydroxyapatite, tricalcium phosphate TCP and osteoconductive growth factors bone morphogenetic protein, BMP, and transforming growth factor, TGFβ.

One or more biocompatible pins 59 may traverse the void 58 disposed between cortical bone portions 51 and 52. One or more rotational fixation mechanisms 56 are located within the void on the one or more pins, the one or more rotational fixation mechanisms including at least one sharp protrusion 57 configured to penetrate a vertebral body. The sharp protrusion may have different cross-sectional profiles.

In an embodiment, the spinal implants and bone grafts provided in FIGS. 1-5 and 9-11 have sharp protrusions that penetrate the vertebral body in a substantially perpendicular plane.

In one embodiment, the method (not claimed) of the present disclosure incorporates use of a da Vinci surgical system to implant the spinal implant into the patient during surgery.

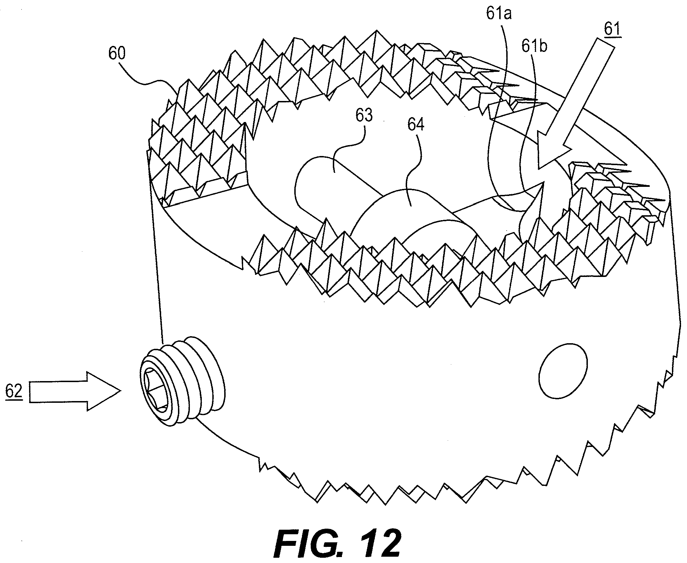

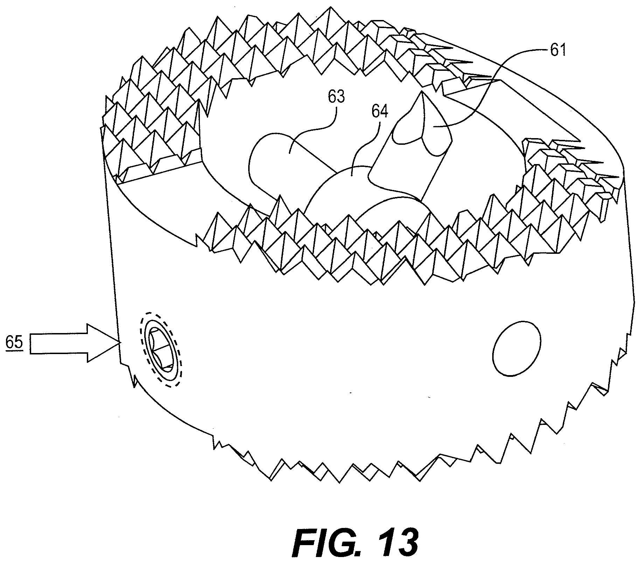

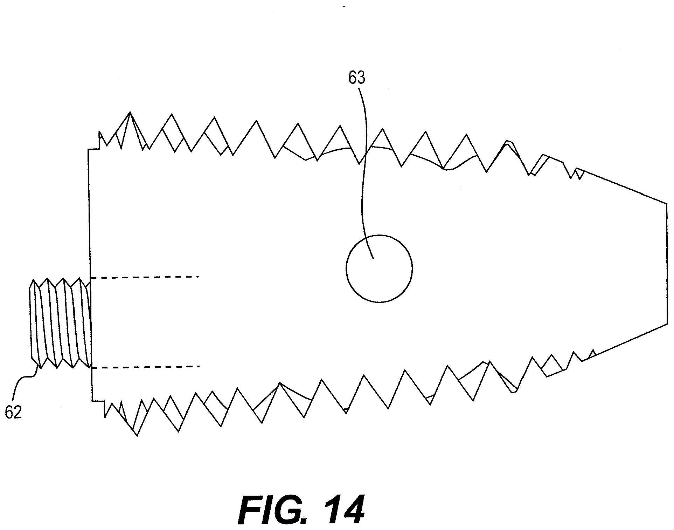

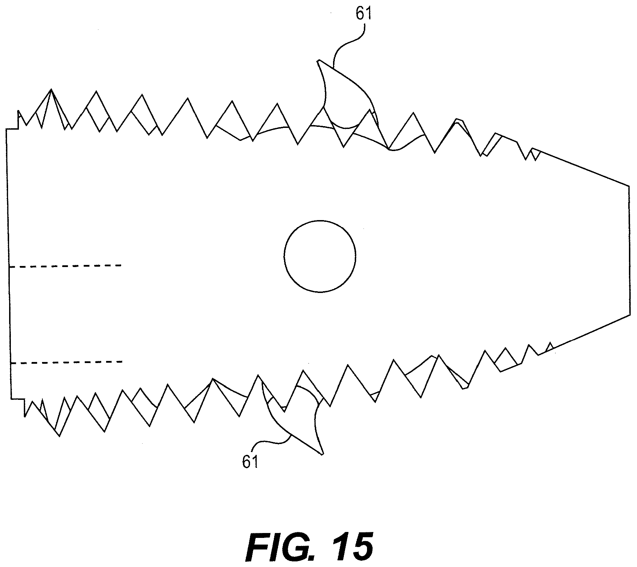

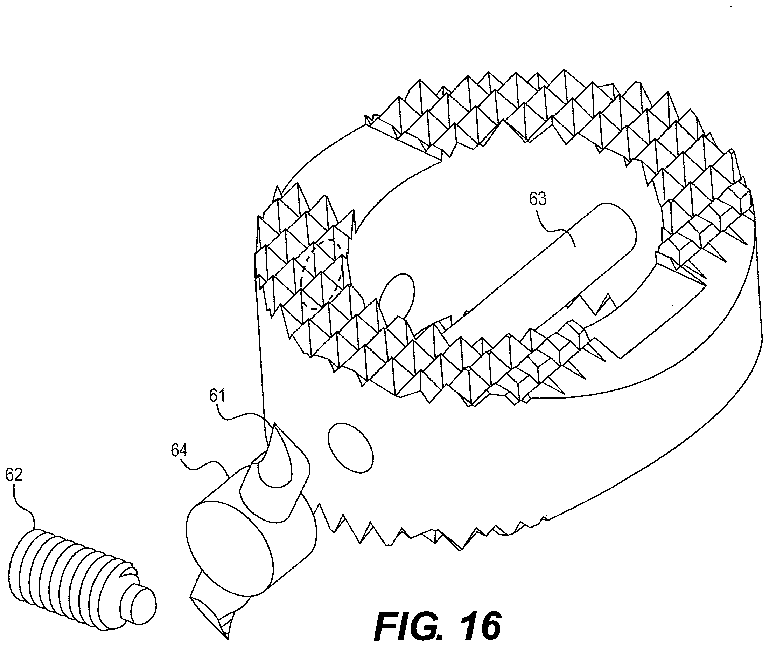

FIGS. 12-16 show different views of a spinal implant 60 of the invention. The spinal implant of this embodiment may be used, for example, in anterior lumbar interbody fusion ALIF procedures. In the embodiments shown in these figures, the sharp protrusion 61, which is connected to the rotational fixation mechanism, may have a double angle. The first angle 61A is sloped at an angle from about 5° to about 35° in the general direction of the protrusion. The second angle 61B is sloped at an angle from about 10° to about 60° in the general direction of the protrusion. It has been discovered that angles within these ranges lead to optimal penetration and lodging into hard tissue. Hexagonal head screw 62 is used in this embodiment to engage with threads 65, pushing the rotational fixation mechanism 64 such that it rotates about an axis e.g., pin 63, urging the sharp protrusion 61 into a vertebral body or other hard tissue. As shown in FIG. 15 , certain embodiments may employ two sharp protrusions, one on the superior portion of the implant and one on the inferior portion of the implant.

In embodiments of the invention, the sharp protrusion may curve inward toward the tip, rather than proceed toward the tip linearly as a result of one or more angles.

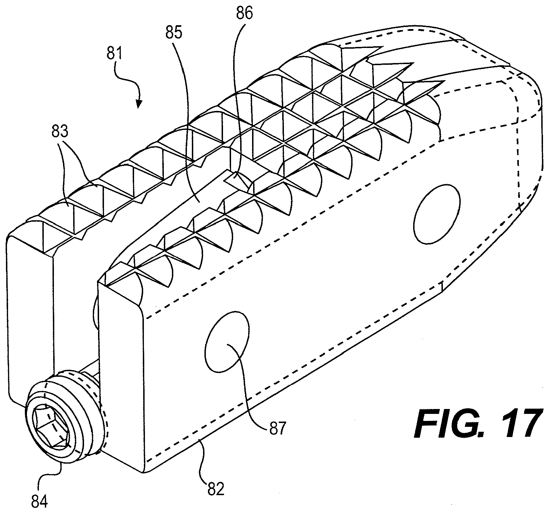

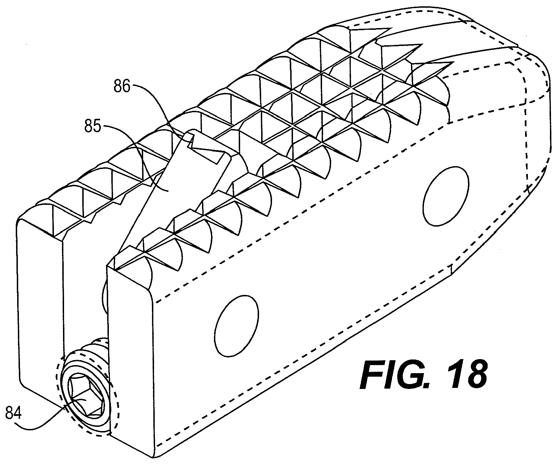

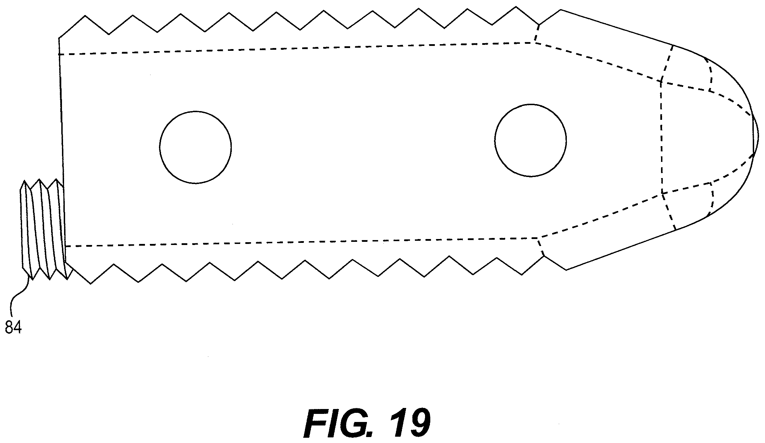

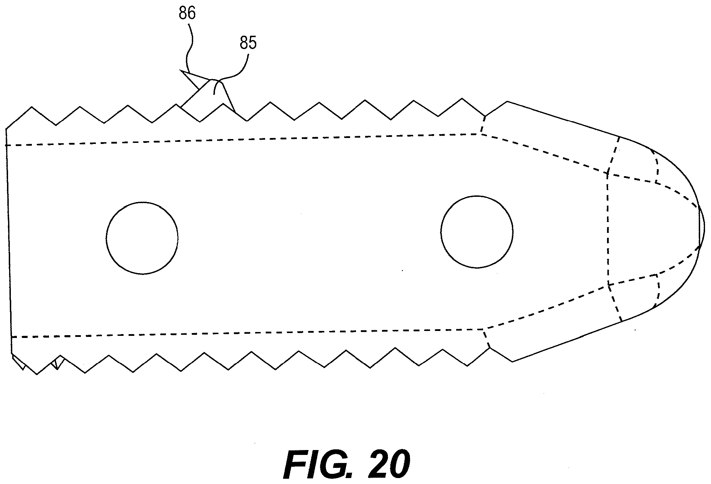

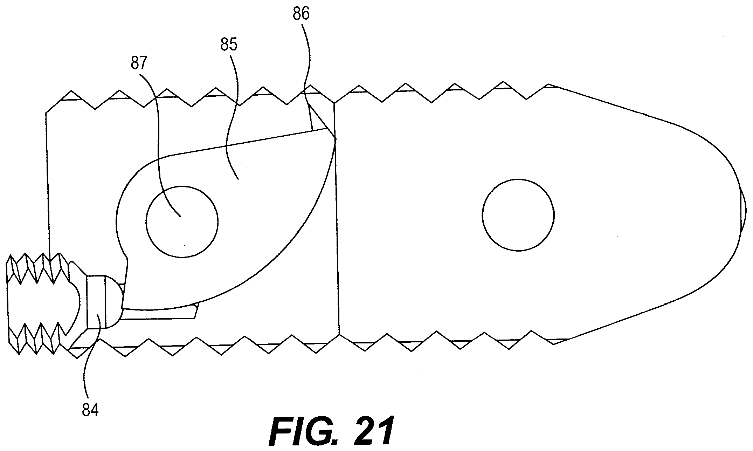

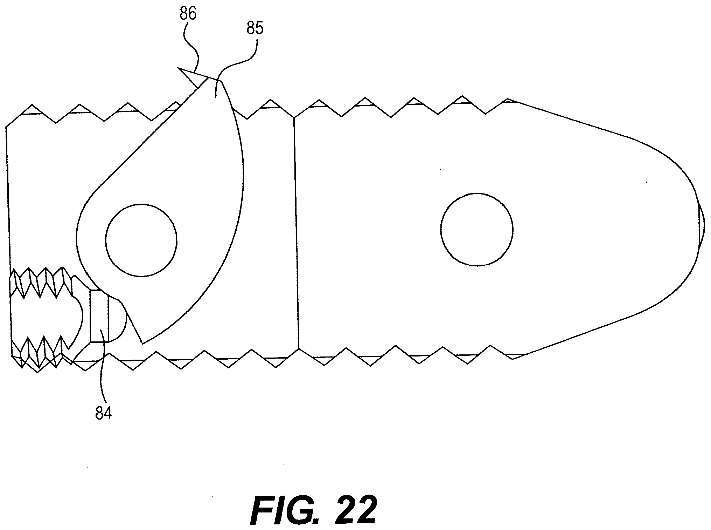

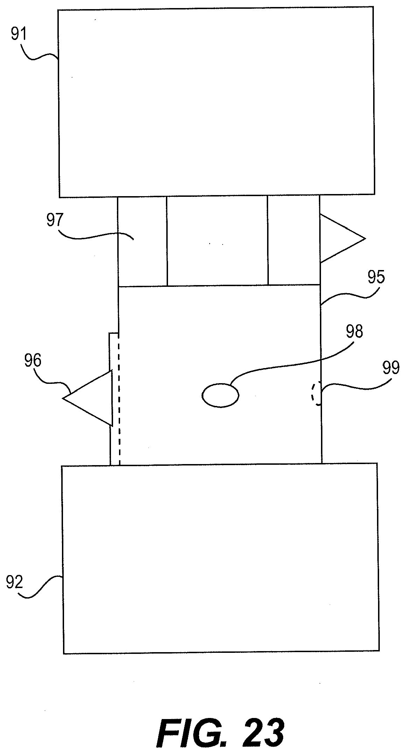

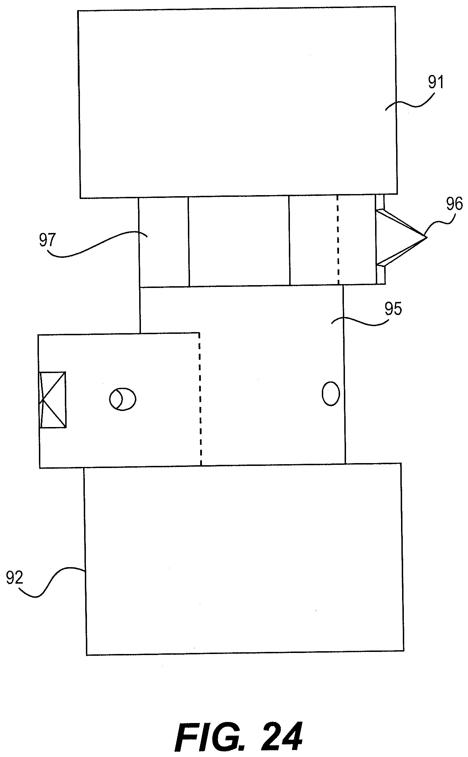

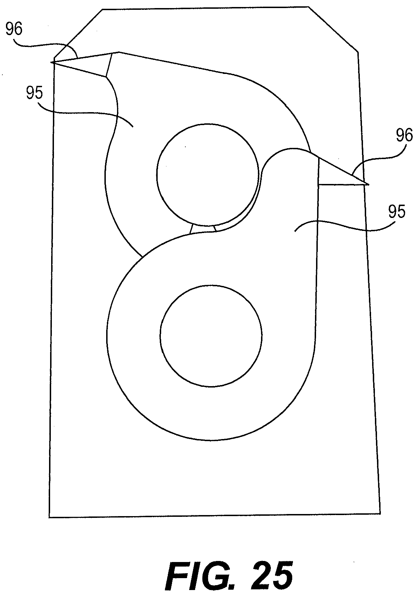

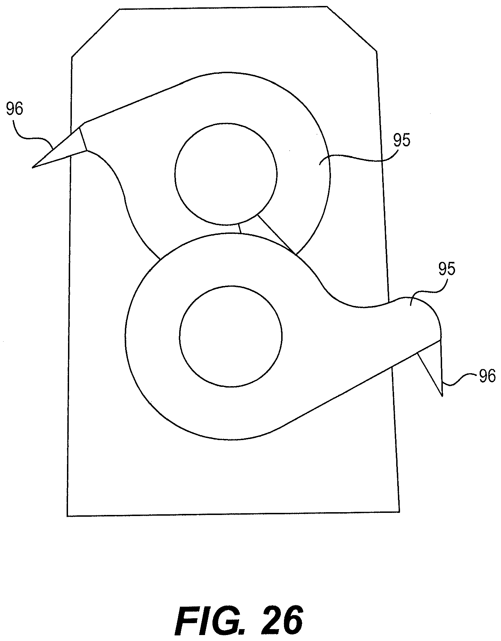

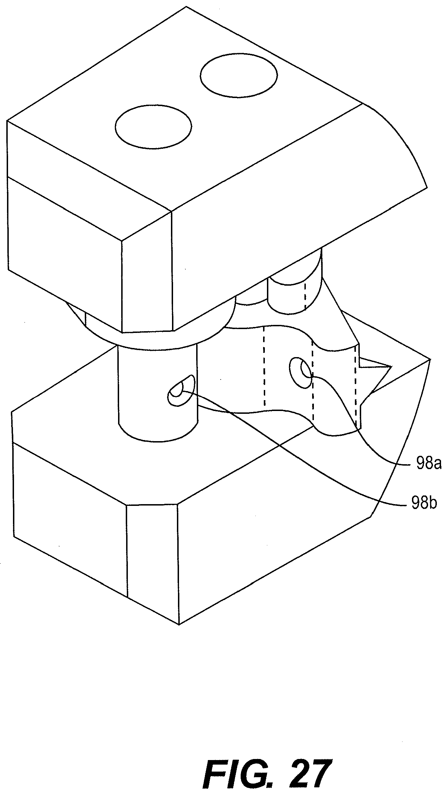

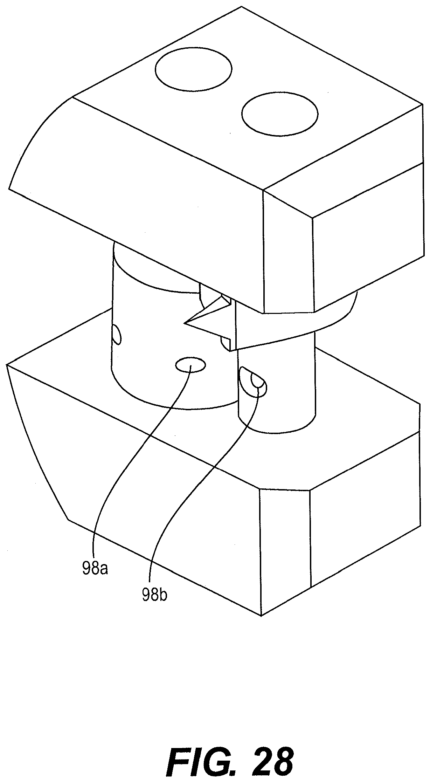

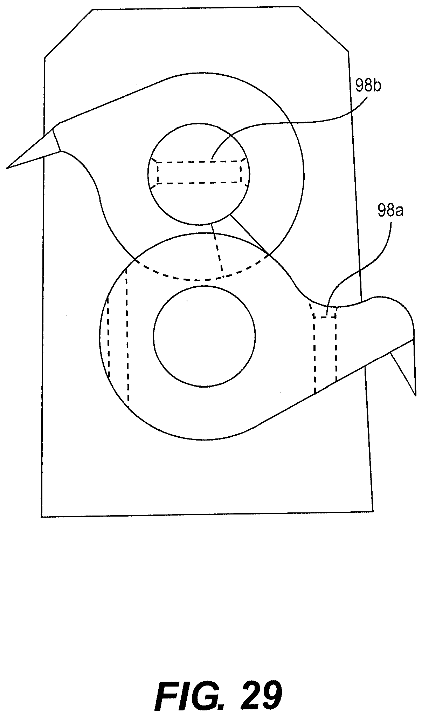

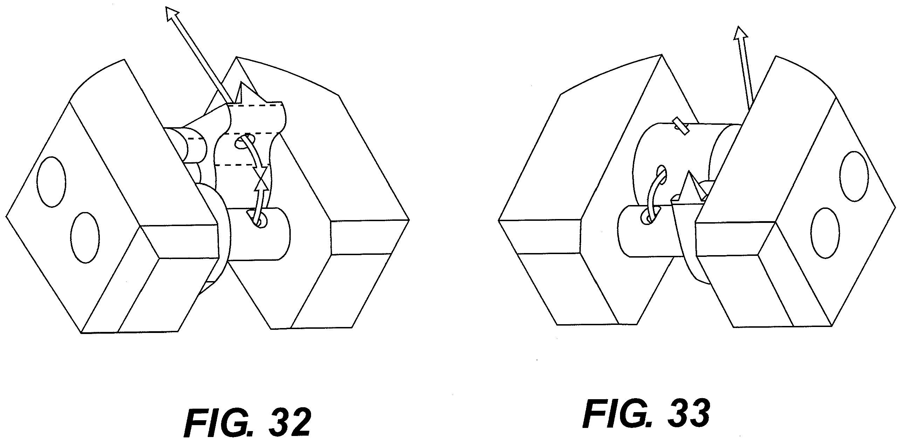

FIGS. 17-22 provide different views of a spinal implant. The spinal implant of this embodiment may be used, for example, in posterior lumbar interbody fusion PLIF procedures. Both the superior surface 81 and the inferior surface 82 are textured 83 and configured to contact a portion of hard tissue e.g., a vertebral body. Screw 84 is used in this embodiment to engage with threads, pushing the rotational fixation mechanism 85 such that it rotates about axis e.g., a pin or rod 87, urging the sharp protrusion 86 into a vertebral body or other hard tissue. FIGS. 23-33 provide different views of a spinal implant of the invention. The spinal implant of this embodiment may be used, for example, in anterior cervical fusion procedures. A first portion 91 and a second portion 92 are provided, each having an inferior and a superior surface. One or more axes e.g., pins or rods 97 may be used to hold together the first and second portion. One or more rotational fixation mechanisms 95 may be located between the first portion and the second portion, substantially centered on an axis. The rotational fixation mechanisms may comprise an opening through which the one or more axes traverse to secure the rotational fixation mechanisms in place. The rotational fixation mechanisms include at least one sharp protrusion 96 configured to penetrate a vertebral body. This is useful for providing stabilization of the spinal area where fusion occurs, and the resultant spinal bone graft may be used without additional stabilizing or anchoring structures, such as supporting plates or screws.

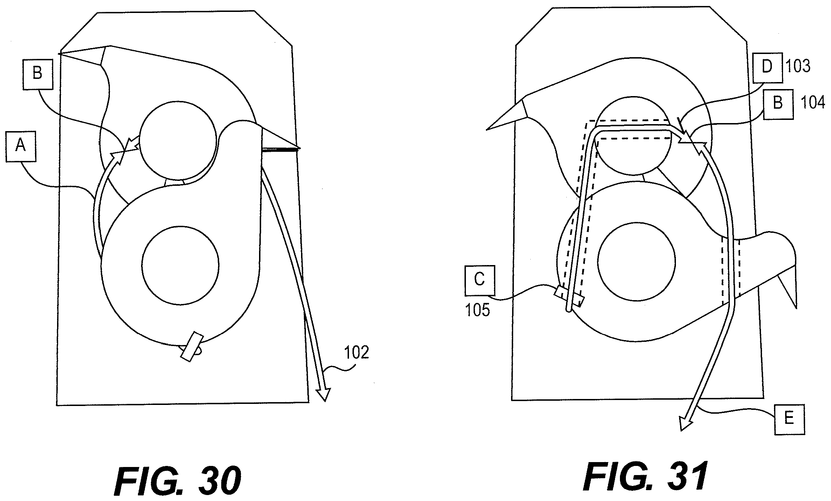

In certain embodiments see e.g., FIGS. 30-33 , a wire 102 is used to engage with the rotational fixation mechanisms, pulling the rotational fixation mechanism 95 to rotate about their axis e.g., pin, urging the sharp protrusion 96 into a vertebral body or other hard tissue. The wire 102 may be made of a variety of materials, including, for example, a composite material such as FiberWire, Kevlar or a carbon fiber weave. The wire 102 may be secured in the end 105 of a rotational fixation mechanism and may be threaded through holes in the rotational fixation mechanisms 98A and axis 98B. The wire contains a directional barb 103 that prevents the rotational fixation mechanisms from reversing direction once the barb 103 is pulled through the opening in the axis. The wire also may have a break point 104 at a designated spot, the break point designed to separate at a predetermined tension in the wire, thus locking the sharp protrusions into position. Upon implantation, a surgeon may pull the wire at the appropriate tension such that the sharp protrusions will penetrate and be lodged into the vertebral bodies. A portion of the wire may be designed to sever at the break point.

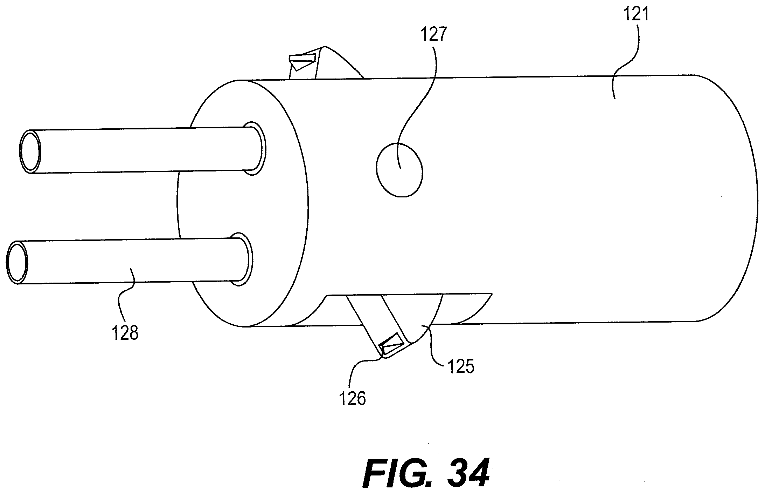

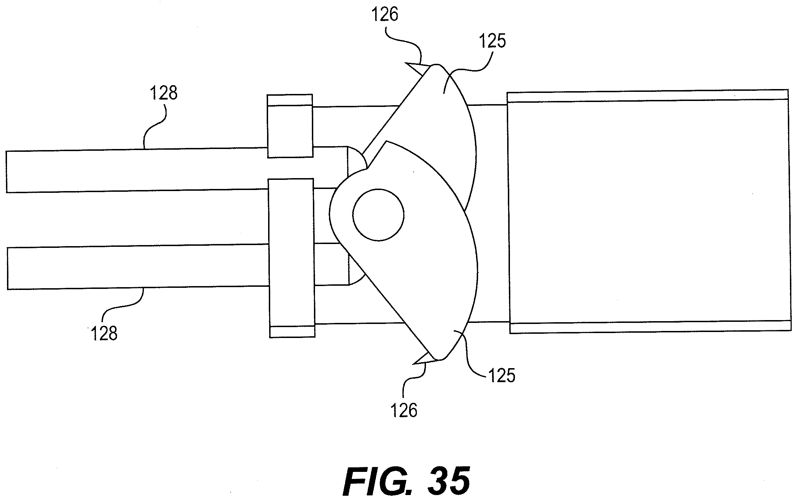

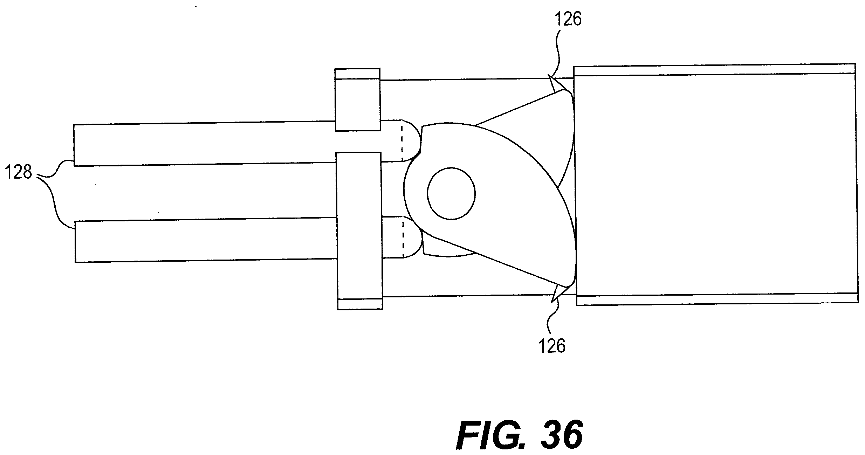

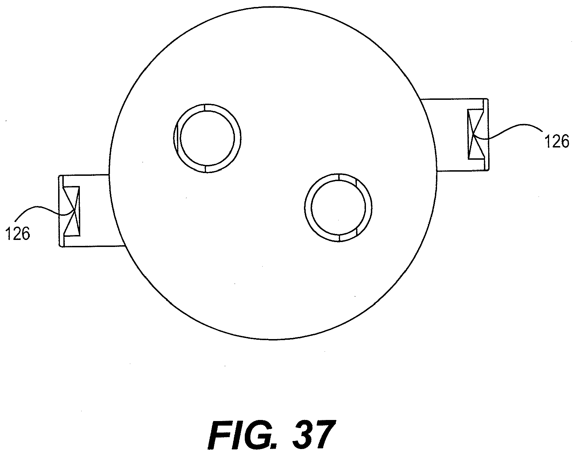

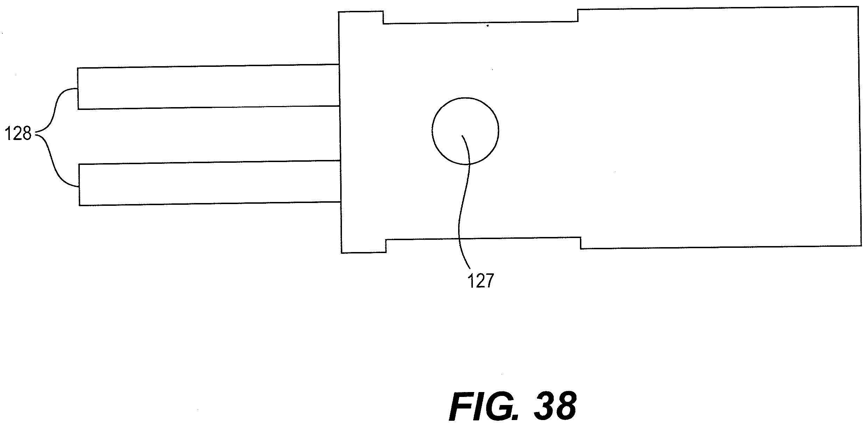

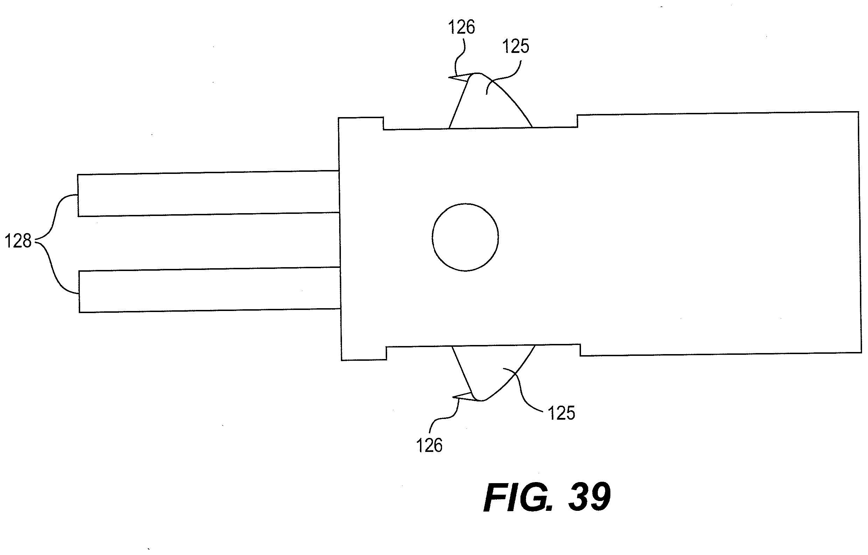

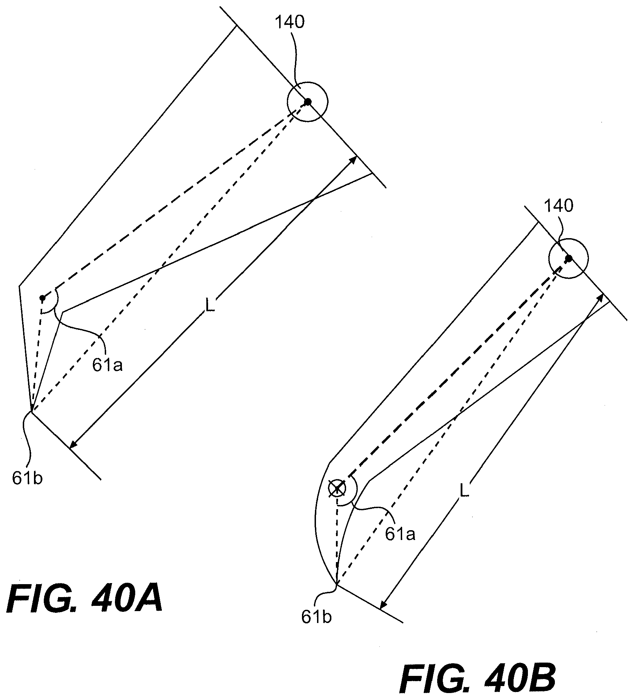

FIGS. 34-39 provide views of a compound medical implant that may be used for fixation and integration with hard tissue at different locations in the body, including, but not limited to, the knee, ankle, hip, shoulder, elbow, fingers and vertebrae. The implant comprises an implant body 121 and rotational fixation mechanisms 125, which rotate around an axis 127 and are attached to sharp protrusions 126. External pins 128 may be used to push the respective rotational fixation mechanisms 125, thus allowing the fixation mechanisms to rotate about their respective axes, urging the sharp protrusions into hard tissue.

The implants of the invention can be used, for example, at a knee joint during an operation such as cruciate ligament reconstruction. In such an operation, the implants of the invention may act as bone plugs, inserted into bone tunnels drilled into the relevant bones surrounding the knee e.g., the tibia or femur. One common approach to cruciate ligament reconstruction is the use of the patellar tendon to form a bone-tendon-bone graft. This involves cutting out a bone block from the top of the patella. The medical implants of the invention may be used in a bone-tendon-bone composite graft for use in cruciate ligament reconstruction along with a tibial drill guide for forming the tibial tunnel, a trefoil rasp for forming channels in the bone tunnels and a bone block drill guide for forming the bone plugs of the graft. In some aspects, a bone tunnel may be formed in each of two bones of the joint. In knee surgery, these are the femur and the tibia. Preferably, the bone tunnel is formed by drilling a core out through the bone such that the core might be used to form the bone plug in the composite graft. The bone plugs are machined to form two longitudinal substantially parallel grooves opposite one another. At least one ligament replacement, such as a semitendinosus tendon, and/or gracilis, may be extended between both of two bone plugs along the parallel grooves in each plug. The ligament replacement may be attached to the two bone plugs. Each bone plug may be inserted into one of the bone tunnels and secured therein by an interference screw. The use of the bone-tendon-bone composite graft of the invention results in a reconstructed cruciate ligament. The aforementioned embodiments may include two or more rotational fixation mechanisms e.g., six, eight or 10, each including a sharp protrusion. The two or more rotational fixation mechanisms may share an axis, or they may be centered on separate axes such that they are in series.

In some applications, it may be difficult for the implants of the invention to penetrate hard tissue e.g., cartilage and cortical bone. Thus, to facilitate penetration of a sharp protrusion into hard tissue e.g., in a vertebral body or a long bone one may use a drill guide with or without a template implant containing a drill hole guide channel. In one apsect, a surgeon may position a drill guide template graft in the appropriate location e.g., the vertebrae between L4 and L5. For example, the drill guide template then may be used to drill a preliminary hole into L4. The drill guide template is then withdrawn prior to insertion of the implant, whereby the sharp protrusion may be maneuvered to penetrate the hard tissue through the pre-drilled preliminary holes.

In embodiments of the invention, the sharp protrusions may include teeth or barbs to prevent reverse migration once the protrusions are lodged into hard tissue.

It is to be understood that the scope of the present invention is not to be limited to the specific embodiments described above. The invention may be practiced other than as particularly described and still be within the scope of the accompanying claims.

## Claims

1. A compound medical implant for fixation and integration with hard bone tissues, the compound medical implant comprising:
a base member (11) including a first portion (1, 91) and a second portion (2, 92), the first portion (1, 91) and the second portion (2, 92) defining a void space, the first portion (1, 91) and the second portion (2, 92) each defined by opposed superior (28) and inferior (29) surfaces configured to contact first and second hard bone tissue of a patient;
one or more rotational fixation mechanisms (64);
one or more biocompatible rods (63, 97) that are permanently attached to the base member (11), wherein:
each biocompatible rod (63, 97) from the one or more biocompatible rods (63, 97) are approximately parallel to the opposed superior (28) and inferior (29) surfaces and traverses the void space from the first portion (1, 91) to the second portion (2, 92),
the one or more biocompatible rods (63, 97) are rotatable, and
each rotational fixation mechanism (64) from the one or more rotational fixation mechanisms (64) configured to rotate around a biocompatible rod (63, 97) from the one or more biocompatible rods (63, 97) and centered on an axis (127) of the biocompatible rod (63, 97) in response to at least one of the one or more rotational fixation mechanisms (64) being pushed by a screw (62) advancing along an axis substantially orthogonal with the axis (127) of the biocompatible rod (63); and
two or more sharp protrusions (61, 96) attached to the one or more rotational fixation mechanisms (64), each sharp protrusion (61, 96) from the two or more sharp protrusions (61) configured to be urged into one of the first or the second hard bone tissue by the one or more rotational fixation mechanisms (64),
a first sharp protrusion (61, 96) of the two or more sharp protrusions (61, 96) having a first proximal end attached to the one or more rotational fixation mechanisms (64) and a first distal end , a second sharp protrusion (61, 96) of the two or more sharp protrusions (61, 96) having a second proximal end attached to the one or more rotational fixation mechanisms (64) and a second distal end ,
wherein:
the first distal end of the first sharp protrusion (61, 96) is configured to penetrate and become lodged into the first hard bone tissue at a first integration site of the first hard bone tissue facing the superior surfaces (28), and
the second distal end of the second sharp protrusion (61, 96) is configured to penetrate and become lodged into the second hard bone tissue at a second integration site of the second hard bone tissue facing the inferior (29) surfaces.

2. The compound medical implant of claim 1, wherein:
the first integration site and the second integration site are located on a vertebra, a knee, a shoulder, an elbow, a wrist, an ankle, a hip, or a finger.

3. The compound medical implant of claim 1, wherein a first rotational fixation mechanism (64) of the one or more rotational fixation mechanisms (64) and the first sharp protrusion (61, 96) are independently made of a member, wherein the member is a cortical bone, a biocompatible metal, a biocompatible polymer, or a biocompatible inorganic material.

4. The compound medical implant of claim 3, wherein the first rotational fixation mechanism (64) and the first sharp protrusion (61, 96) are each made of the cortical bone.

5. The compound medical implant of claim 1, wherein the first sharp protrusion (61, 96) has a length ranging from 0.5 mm to 8 mm.

6. The compound medical implant of claim 1, wherein the first sharp protrusion (61, 96) has a pyramidal cross section.

7. The compound medical implant of claim 1, wherein the first distal end of the first sharp protrusion (61, 96) has a first angle and a second angle.

8. The compound medical implant of claim 7, wherein:
the first angle is sloped at an angle from 5° to 35° in a first direction of the sharp protrusion (61, 96) penetration, and
the second angle is sloped at an angle from 10° to 60° in a second direction of the sharp protrusion (61, 96) penetration.

9. The compound medical implant of claim 1, wherein each of the opposed superior (28) and inferior (29) surfaces include a plurality of hard bone tissue engaging protrusions.

10. The compound medical implant of claim 9, wherein:
each of the opposed superior (28) and inferior (29) surfaces are textured (83), and
the first sharp protrusion (61, 96) exceeds a height of the textured (83) superior and inferior (29) surfaces.

11. The compound medical implant of claim 1, further comprising one or more screws configured to push the one or more rotational fixation mechanisms 64) such that the one or more rotational fixation mechanisms (64) rotate about the axis (127) of the biocompatible rod (63), wherein rotation of the one or more rotational fixation mechanisms (64) is less than rotation of the one or more screws.

12. The compound medical implant of claim 11, wherein the one or more screws are orthogonally oriented to the axis (127).

## Patentansprüche

1. Ein zusammengesetztes medizinisches Implantat zur Fixierung und Integration in hartes Knochengewebe, wobei das zusammengesetzte medizinische Implantat umfasst:
ein Basiselement (11) mit einem ersten Abschnitt (1, 91) und einem zweiten Abschnitt (2, 92), wobei der erste Abschnitt (1, 91) und der zweite Abschnitt (2, 92) einen Hohlraum begrenzen, wobei der erste Abschnitt (1, 91) und der zweite Abschnitt (2, 92) jeweils durch einander gegenüberliegende obere (28) und untere (29) Flächen definiert sind, die so ausgebildet sind, dass sie mit erstem und zweitem hartem Knochengewebe eines Patienten in Kontakt kommen;
einen oder mehrere Rotationsfixierungsmechanismen (64);
eine oder mehrere biokompatible Stäbe (63, 97), die dauerhaft an dem Basiselement (11) befestigt sind, wobei:
jede der einen oder mehreren biokompatiblen Stäbe (63, 97) annähernd parallel zu den gegenüberliegenden oberen (28) und unteren (29) Flächen verläuft und den Hohlraum vom ersten Abschnitt (1, 91) zum zweiten Abschnitt (2, 92) durchquert,
die eine oder mehreren biokompatiblen Stäbe (63, 97) drehbar sind, und
jeder Drehfixierungsmechanismus (64) aus den einen oder mehreren Drehfixierungsmechanismen (64) so konfiguriert ist, dass er sich um einen biokompatiblen Stab (63, 97) aus den einen oder mehreren biokompatiblen Stäben (63, 97) und zentriert auf eine Achse (127) des biokompatiblen Stabs (63, 97) zu drehen, als Reaktion darauf, dass mindestens einer der einen oder mehreren Drehfixierungsmechanismen (64) von einer Schraube (62) gedrückt wird, die sich entlang einer Achse vorwärtsbewegt, die im Wesentlichen orthogonal zur Achse (127) des biokompatiblen Stabs (63) verläuft; und
zwei oder mehr scharfe Vorsprünge (61, 96), die an dem einen oder den mehreren Rotationsbefestigungsmechanismen (64) angebracht sind, wobei jeder scharfe Vorsprung (61, 96) von den zwei oder mehr scharfen Vorsprüngen (61) so ausgebildet ist, dass er durch den einen oder die mehreren Rotationsbefestigungsmechanismen (64) in das erste oder das zweite harte Knochengewebe gedrückt wird,
wobei ein erster spitzer Vorsprung (61, 96) der zwei oder mehr spitzen Vorsprünge (61, 96) ein erstes proximales Ende aufweist, das an dem einen oder den mehreren Rotationsfixierungsmechanismen (64) befestigt ist, sowie ein erstes distales Ende, und ein zweiter spitzer Vorsprung (61, 96) der zwei oder mehr spitzen Vorsprünge (61, 96) ein zweites proximales Ende aufweist, das an dem einen oder den mehreren Rotationsfixierungsmechanismen (64) befestigt ist, sowie ein zweites distales Ende,
wobei:
das erste distale Ende des ersten scharfen Vorsprungs (61, 96) so ausgebildet ist, dass es in das erste harte Knochengewebe an einer ersten Verankerungsstelle des ersten harten Knochengewebes, die den oberen Flächen (28) zugewandt ist, eindringt und sich darin festsetzt, und
das zweite distale Ende des zweiten scharfen Vorsprungs (61, 96) so ausgebildet ist, dass es in das zweite harte Knochengewebe an einer zweiten Integrationsstelle des zweiten harten Knochengewebes, die den unteren Oberflächen (29) zugewandt ist, eindringt und sich darin festsetzt.

2. Das zusammengesetzte medizinische Implantat nach Anspruch 1, wobei:
die erste Verankerungsstelle und die zweite Verankerungsstelle sich an einem Wirbel, einem Knie, einer Schulter, einem Ellenbogen, einem Handgelenk, einem Sprunggelenk, einer Hüfte oder einem Finger befinden.

3. Das zusammengesetzte medizinische Implantat nach Anspruch 1, wobei ein erster Rotationsfixierungsmechanismus (64) der einen oder mehreren Rotationsfixierungsmechanismen (64) und der erste scharfe Vorsprung (61, 96) unabhängig voneinander aus einem Element bestehen, wobei das Element aus Kortikalknochen, einem biokompatiblen Metall, einem biokompatiblen Polymer oder einem biokompatiblen anorganischen Material besteht.

4. Das zusammengesetzte medizinische Implantat nach Anspruch 3, wobei der erste Rotationsfixierungsmechanismus (64) und der erste scharfe Vorsprung (61, 96) jeweils aus dem Kortikalisknochen bestehen.

5. Das zusammengesetzte medizinische Implantat nach Anspruch 1, wobei der erste scharfe Vorsprung (61, 96) eine Länge im Bereich von 0,5 mm bis 8 mm aufweist.

6. Das zusammengesetzte medizinische Implantat nach Anspruch 1, wobei der erste scharfe Vorsprung (61, 96) einen pyramidenförmigen Querschnitt aufweist.

7. Das zusammengesetzte medizinische Implantat nach Anspruch 1, wobei das erste distale Ende des ersten scharfen Vorsprungs (61, 96) einen ersten Winkel und einen zweiten Winkel aufweist.

8. Das zusammengesetzte medizinische Implantat nach Anspruch 7, wobei:
der erste Winkel in einer ersten Richtung des Eindringens des scharfen Vorsprungs (61, 96) in einem Winkel von 5° bis 35° geneigt ist und
der zweite Winkel in einer zweiten Richtung des Eindringens des scharfen Vorsprungs (61, 96) in einem Winkel von 10° bis 60° geneigt ist.

9. Das zusammengesetzte medizinische Implantat nach Anspruch 1, wobei sowohl die einander gegenüberliegende obere (28) als auch die untere (29) Oberfläche jeweils eine Vielzahl von Vorsprüngen aufweisen, die in hartes Knochengewebe eingreifen.

10. Das zusammengesetzte medizinische Implantat nach Anspruch 9, wobei:
sowohl die einander gegenüberliegende obere (28) als auch die untere (29) Oberfläche strukturiert (83) sind, und
der erste scharfe Vorsprung (61, 96) die Höhe der strukturierten (83) oberen und unteren (29) Oberflächen übersteigt.

11. Das zusammengesetzte medizinische Implantat nach Anspruch 1, das ferner eine oder mehrere Schrauben umfasst, die so konfiguriert sind, dass sie den einen oder die mehreren Rotationsfixierungsmechanismen (64) so verschieben, dass sich der eine oder die mehreren Rotationsfixierungsmechanismen (64) um die Achse (127) des biokompatiblen Stabs (63) drehen, wobei die Drehung des einen oder der mehreren Rotationsfixierungsmechanismen (64) geringer ist als die Drehung der einen oder mehreren Schrauben.

12. Das zusammengesetzte medizinische Implantat nach Anspruch 11, wobei die eine oder mehreren Schrauben orthogonal zur Achse (127) ausgerichtet sind.

## Revendications

1. Implant médical composite destiné à la fixation et à l'intégration dans les tissus osseux durs, ledit implant médical composite comprenant :
un élément de base (11) comprenant une première partie (1, 91) et une deuxième partie (2, 92), la première partie (1, 91) et la deuxième partie (2, 92) définissant un espace vide, la première partie (1, 91) et la deuxième partie (2, 92) étant chacune définies par des surfaces supérieure (28) et inférieure (29) opposées, configurées pour entrer en contact avec des premier et deuxième tissus osseux durs d'un patient ;
un ou plusieurs mécanismes de fixation par rotation (64) ;
une ou plusieurs tiges biocompatibles (63, 97) qui sont fixées de manière permanente à l'élément de base (11), dans lequel :
chaque tige biocompatible (63, 97) parmi la ou les tiges biocompatibles (63, 97) est approximativement parallèle aux surfaces supérieure (28) et inférieure (29) opposées et traverse l'espace vide de la première partie (1, 91) à la deuxième partie (2, 92),
la ou les tiges biocompatibles (63, 97) sont rotatives, et
chaque mécanisme de fixation rotatif (64) parmi le ou les mécanismes de fixation rotatifs (64) est configuré pour tourner autour d'une tige biocompatible (63, 97) parmi la ou les tiges biocompatibles (63, 97) et centré sur un axe (127) de la tige biocompatible (63, 97) en réponse au fait qu'au moins l'un desdits un ou plusieurs mécanismes de fixation rotatifs (64) est poussé par une vis (62) avançant le long d'un axe sensiblement orthogonal à l'axe (127) de la tige biocompatible (63) ; et
deux ou plusieurs saillies acérées (61, 96) fixées au ou aux mécanismes de fixation rotatifs (64), chaque saillie acérée (61, 96) parmi les deux ou plusieurs saillies acérées (61) étant configurée pour être enfoncée dans l'un des premier ou deuxième tissus osseux durs par le ou les mécanismes de fixation rotatifs (64),
une première saillie pointue (61, 96) parmi les deux ou plusieurs saillies pointues (61, 96) comportant une première extrémité proximale fixée au ou aux mécanismes de fixation rotatifs (64) et une première extrémité distale , une deuxième saillie pointue (61, 96) parmi les deux ou plusieurs saillies pointues (61, 96) comportant une deuxième extrémité proximale fixée au ou aux mécanismes de fixation rotatifs (64) et une deuxième extrémité distale ,
dans lequel :
la première extrémité distale de la première saillie pointue (61, 96) est configurée pour pénétrer et se loger dans le premier tissu osseux dur au niveau d'un premier site d'intégration dudit premier tissu osseux dur faisant face aux surfaces supérieures (28), et
la deuxième extrémité distale de la deuxième saillie pointue (61, 96) est configurée pour pénétrer et se loger dans le deuxième tissu osseux dur au niveau d'un deuxième site d'intégration dudit deuxième tissu osseux dur faisant face aux surfaces inférieures (29).

2. Implant médical composite selon la revendication 1, dans lequel :
le premier site d'intégration et le deuxième site d'intégration sont situés sur une vertèbre, un genou, une épaule, un coude, un poignet, une cheville, une hanche ou un doigt.

3. Implant médical composite selon la revendication 1, dans lequel un premier mécanisme de fixation rotatif (64) parmi le ou les mécanismes de fixation rotatifs (64) et la première saillie acérée (61, 96) sont chacun constitués d'un élément, cet élément étant un os cortical, un métal biocompatible, un polymère biocompatible ou un matériau inorganique biocompatible.

4. Implant médical composite selon la revendication 3, dans lequel le premier mécanisme de fixation en rotation (64) et la première saillie acérée (61, 96) sont chacun constitués d'os cortical.

5. Implant médical composite selon la revendication 1, dans lequel la première saillie acérée (61, 96) présente une longueur comprise entre 0,5 mm et 8 mm.

6. Implant médical composite selon la revendication 1, dans lequel la première saillie acérée (61, 96) présente une section transversale pyramidale.

7. Implant médical composite selon la revendication 1, dans lequel la première extrémité distale de la première saillie acérée (61, 96) présente un premier angle et un deuxième angle.

8. Implant médical composite selon la revendication 7, dans lequel :
le premier angle est incliné selon un angle compris entre 5° et 35° dans une première direction de pénétration de la saillie acérée (61, 96), et
le deuxième angle est incliné selon un angle compris entre 10° et 60° dans une deuxième direction de pénétration de la saillie acérée (61, 96).

9. Implant médical composite selon la revendication 1, dans lequel chacune des surfaces supérieure (28) et inférieure (29) opposées comprend une pluralité de saillies s'engageant dans le tissu osseux dur.

10. Implant médical composite selon la revendication 9, dans lequel :
chacune des surfaces supérieure (28) et inférieure (29) opposées est texturée (83), et
la première saillie acérée (61, 96) dépasse la hauteur des surfaces supérieure et inférieure (29) texturées (83).

11. Implant médical composite selon la revendication 1, comprenant en outre une ou plusieurs vis configurées pour pousser le ou les mécanismes de fixation rotatifs (64) de telle sorte que le ou les mécanismes de fixation rotatifs (64) tournent autour de l'axe (127) de la tige biocompatible (63), dans lequel la rotation du ou des mécanismes de fixation rotatifs (64) est inférieure à la rotation de la ou des vis.

12. Implant médical composite selon la revendication 11, dans lequel la ou les vis sont orientées orthogonalement par rapport à l'axe (127).
